(19) **Europäisches Patentamt / European Patent Office / Office européen des brevets**

(11) **EP 3 503 956 B1**

(12) **EUROPEAN PATENT SPECIFICATION**

(45) Date of publication and mention of the grant of the patent:
**15.11.2023 Bulletin 2023/46**

(21) Application number: **17749574.4**

(22) Date of filing: **28.07.2017**

(51) International Patent Classification (IPC):
**A61B 17/00** (2006.01)    **A61M 25/00** (2006.01)
**A61F 2/24** (2006.01)

(52) Cooperative Patent Classification (CPC):
**A61M 25/0026; A61B 17/00234; A61M 25/0032;**
A61B 2017/00243; A61B 2017/00323; A61F 2/2436;
A61F 2/2439; A61M 25/005; A61M 2025/0034;
A61M 2025/0035; A61M 2025/0036;
A61M 2025/004; A61M 2025/0046;
A61M 2025/0047

(86) International application number:
**PCT/US2017/044470**

(87) International publication number:
**WO 2018/044448 (08.03.2018 Gazette 2018/10)**

(54) **MULTILUMEN CATHETER**

MEHRLUMIGER KATHETER

CATHÉTER À LUMIÈRES MULTIPLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.08.2016 US 201662380873 P**
**27.07.2017 US 201715662093**

(43) Date of publication of application:
**03.07.2019 Bulletin 2019/27**

(73) Proprietor: **Cephea Valve Technologies, Inc.**
**Santa Clara, CA 95054 (US)**

(72) Inventors:
• **VON OEPEN Randolf**
  **Aptos, CA 95003 (US)**
• **MCNIVEN Sean A.**
  **menlo Parek, CA 94025 (US)**
• **VALENCIA Francisco**
  **East Palo Alto, CA 94303 (US)**

(74) Representative: **Boult Wade Tennant LLP**
**Salisbury Square House**
**8 Salisbury Square**
**London EC4Y 8AP (GB)**

(56) References cited:
EP-A1- 1 980 288      EP-A1- 2 529 701
WO-A1-2012/020521     WO-A1-2013/006282
WO-A1-2016/022797     US-A- 4 406 656
US-A- 4 728 319       US-A- 5 472 423
US-A1- 2004 049 207   US-A1- 2004 147 826
US-A1- 2005 277 876   US-A1- 2007 060 997
US-A1- 2007 173 757   US-A1- 2008 188 850
US-B1- 6 180 059

• TAKIZAWA H ET AL: "Development of a microfine active bending catheter equipped with MIF tactile sensors", MICRO ELECTRO MECHANICAL SYSTEMS, 1999. MEMS '99. TWELFTH IEEE INTERNA TIONAL CONFERENCE ON ORLANDO, FL, USA 17-21 JAN. 1999, PISCATAWAY, NJ, USA,IEEE, US, 17 January 1999 (1999-01-17), pages 412-417, XP010321677, ISBN: 978-0-7803-5194-3

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE DISCLOSURE

Intravascular medical procedures allow the performance of therapeutic treatments in a variety of locations within a patient's body while requiring only relatively small access incisions. An intravascular procedure may, for example, eliminate the need for open-heart surgery, reducing risks, costs, and time associated with an open-heart procedure. The intravascular procedure also enables faster recovery times with lower associated costs and risks of complication. An example of an intravascular procedure that significantly reduces procedure and recovery time and cost over conventional open surgery is a heart valve replacement or repair procedure. An artificial valve is guided to the heart through the patient's vasculature. For example, a catheter is inserted into the patient's vasculature and directed to the inferior vena cava. The catheter is then urged through the inferior vena cava toward the heart by applying force longitudinally to the catheter. Upon entering the heart from the inferior vena cava, the catheter enters the right atrium. The distal end of the catheter may be deflected by one or more wires positioned inside the catheter. Precise control of the distal end of the catheter allows for more reliable and faster positioning of a medical device and/or implant and other improvements in the procedure.

The devices can also be directed through the valve chordae or papillary muscles, for example, for interventional therapy to the mitral valve. When such procedures require the use of more than one instrument, each instrument would be dependent upon proper positioning in relation to the valve. Therefore, positioning or steering mechanisms need to be built into each instrument. This adds further cost, complexity, and time to the procedures.

Other procedures may include tracking a catheter and/or access sheath from a puncture in the femoral vein through the intra-atrial septum to the left atrium. This pathway may be used to access the left atrium for ablation of the atrium wall or ablation around the pulmonary veins. Such interventional therapies would require precise alignment with target areas for proper ablation placement. Additionally, alternative access routes and/or access routes to other cavities may be desired.

In US 2005/0277876 there is described a steering system that can be attached to a catheter, guidewire or obturator that consists of polymeric tubing with a center lumen and at least three off-axis lumens evenly spaced around the circumference of the device shaft. The distal sections of the off-axis lumens are formed to induce curvature of the tip of the device by a physician controlled pressure source, which may be foot activated. Forming the individual lumens to induce curvature of the shaft of the device in a certain direction is done by making one side of each lumen significantly longer, such as with a one sided corrugated configuration. These arrangements induce curvature in predetermined directions when pressurized.

In US 2004/0049207 there are described devices, systems and methods for tissue approximation and repair at treatment sites. The devices, systems and methods are said to find use in a variety of therapeutic procedures, including endovascular, minimally-invasive, and open surgical procedures, and to be capable of use in various anatomical regions, including the abdomen, thorax, cardiovascular system, heart, intestinal tract, stomach, urinary tract, bladder, lung, and other organs, vessels, and tissues. The described devices, systems and methods are said to be particularly useful in those procedures requiring minimally-invasive or endovascular access to remote tissue locations, where the instruments utilized must negotiate long, narrow, and tortuous pathways to the treatment site. In addition, many of the devices and systems are adapted to be reversible and removable from the patient at any point without interference with or trauma to internal tissues.

In WO 2016/022797 there are described multi-lumen cannulae that can be used in various different medical procedures. The multi-lumen cannulae can comprise an elongated body comprising multiple different ports that connect to the various different sidewall lumens contained within the elongated body. The multi-lumen cannulae can also comprise a central lumen that extends through the entire elongated body and can be fluidly connected to the various different sidewall lumens. The multi-lumen cannulae can further comprise two balloons on the exterior of the elongated body, which can be used to isolate the right atrium of a patient's heart.

In US 2004/0147826 there is described a guiding catheter system that employs an outer jacket with one or more inflation lumens provided along the length of the jacket. The lumens are in fluid communication with one or more fluted balloons mounted at a distal end of the outer jacket. A torquable stylet is movably disposed within an open lumen of the outer jacket. A guidewire is movably disposed in an open lumen of the torquable stylet. The outer jacket can be provided with break-away features allowing the jacket to be peeled off of a pacing lead.

In US 5,472,423 there is described a flexible catheter for use with an antenna which radiates high-frequency or microwave energy, the catheter having a carrier tube and an outer layer of microporous polytetrafluoroethylene on the outer circumference of the carrier tube.

In US 2007/0060997 there is described a steerable catheter having a flexible shaft extending between a proximal and distal end, with a hub affixed to the proximal end, and at least one actuator for causing the catheter distal end to move or bend in a desired direction. The catheter shaft defines at least one passage or steering lumen, at least one of which has a closed distal end. The hub may have a number of actuators, such that each actuator may affect the pressure of a pressure medium in a steering lumen. When specific actuators are activated, the pressures in each of the steering lumens may be disparately raised and/or lowered, so as to cause a portion of the catheter shaft to tend to bend in a specific direction. For example, one or some of the actuators may be used to change the pressure of the associated steering lumens, causing the catheter shaft to bend. Also, in the case of a steerable catheter having more than one actuator, all of the actuators may be activated to raise the pressure of all of the steering lumens, tending to temporarily increase the column stiffness and pushability of the catheter shaft.

In WO 2012/020521 there is described a catheter provided with: a tube member; a pair of bar springs provided inside the tube member along the central axis thereof, one of the bar springs being offset from the central axis and the other being offset from the central axis in the opposite direction; control-wire insertion passages provided inside the tube member along the central axis thereof, located respectively on one side and the other side of a virtual plane that passes through the central axes of the two bar springs; and control wires slidably inserted into the control-wire insertion passages, one end of each control wire being connected near the distal end of the tube member.

In WO 2013/006282 there is described a steerable delivery catheter which includes an outer sheath with a housing disposed at a distal end thereof to define a chamber for a medical device, the chamber having an open distal end and a proximal inner shelf. An inner shaft is slidably disposed within the sheath and has an abutment attached to the distal end of the shaft, the abutment being slidably contained within the chamber. During navigation of the catheter, when the inner shaft is tensioned the abutment engages the shelf to apply a compressive force to the sheath to selectively deflect a distal region of the catheter. When the catheter has been navigated to the deployment site the sheath is withdrawn while the abutment maintains the medical device in place as it is deployed.

In EP 2,529,701 there is described a deployment system for deploying an expandable cardiac valve prosthesis. The deployment system comprises a first tube designed to carry an expandable cardiac valve prosthesis. A tip is firmly connected to the first tube at a distal end of the first tube, wherein the tip is designed such, that it detachably accommodates and holds a distal end of a cardiac valve prosthesis. A sheath is designed to be disposed over and hold the prosthesis in a compressed state, and a first actuating mechanism is linked to the sheath and slidable in a proximal direction for stepwise retracting the sheath. The deployment system further comprises a biasing element linked to the tip, and a blocking mechanism, wherein the blocking mechanism is designed such that a movement of the first actuating mechanism in the proximal direction relative to the tip is limitable to a maximum path of travel by the blocking mechanism.

BRIEF SUMMARY OF THE DISCLOSURE

According to the present invention there is provided an intravascular device delivery system. The system comprises an elongated member with a proximal end, a distal end, and a longitudinal axis therebetween, the elongated member being configured to have an intravascular device positioned at and/or connected to the distal end. The elongated member includes a multilumen catheter having a catheter wall having an outer diameter (OD), an internal diameter (ID), and with a plurality of lumens formed within the catheter wall. The plurality of lumens extend from a proximal end of the elongated member to a distal end of the elongated member. At least one of the lumens of the plurality of lumens has an elliptical cross-section and at least one other lumen of the plurality of lumens has a round cross-section. A lumen ratio, equal to the percentage of the cross sectional area of the catheter wall, in a plane perpendicular to the longitudinal axis, between the outer diameter (OD) and the inner diameter (ID), that is taken up by the combined cross-sectional areas of the plurality of lumens, is greater than 30%.

This summary is provided to introduce a selection of concepts that are further described below in the detailed description. This summary is not intended to be used in limiting the scope of the claimed subject matter.

Additional features will be set forth in the description which follows. These and other features will become more fully apparent from the following description and appended claims, or may be learned by the practice of such exemplary arrangements as set forth hereinafter.

BRIEF DESCRIPTION OF THE DRAWING

In order to describe the manner in which the above-recited and other features of the disclosure can be obtained, a more particular description will be rendered by reference to specific examples thereof which are illustrated in the appended drawings. For better understanding, the like elements have been designated by like reference numbers throughout the various accompanying figures. While some of the drawings may be schematic or exaggerated representations of concepts, at least some of the drawings may be drawn to scale. Understanding that the drawings depict some exemplary arrangements, the arrangements will be described and explained with additional specificity and detail through the use of the accompanying drawings in which:

FIG. 1 is a schematic representation of an intravascular device delivery system;

FIG. 2 is a detail perspective view of one example of a distal end of the intravascular device delivery system of FIG. 1;

FIG. 3 is a cross-sectional side view of one example of an elongated member of the intravascular device delivery system of FIG. 1;

FIG. 4 is a detail transverse cross-sectional view of one example of a multilumen catheter of the intravascular device delivery system of FIG. 1;

FIG. 5 is a detail transverse cross-sectional side view of an example of a multilumen catheter of an intravascular device delivery system in accordance with the invention;

FIG. 6 is detail transverse cross-sectional side view of another example of a multilumen catheter of an intravascular device delivery system; and

FIG. 7 is detail transverse cross-sectional side view of another example of a multilumen catheter of an intravascular device delivery system in accordance with the invention.

DETAILED DESCRIPTION

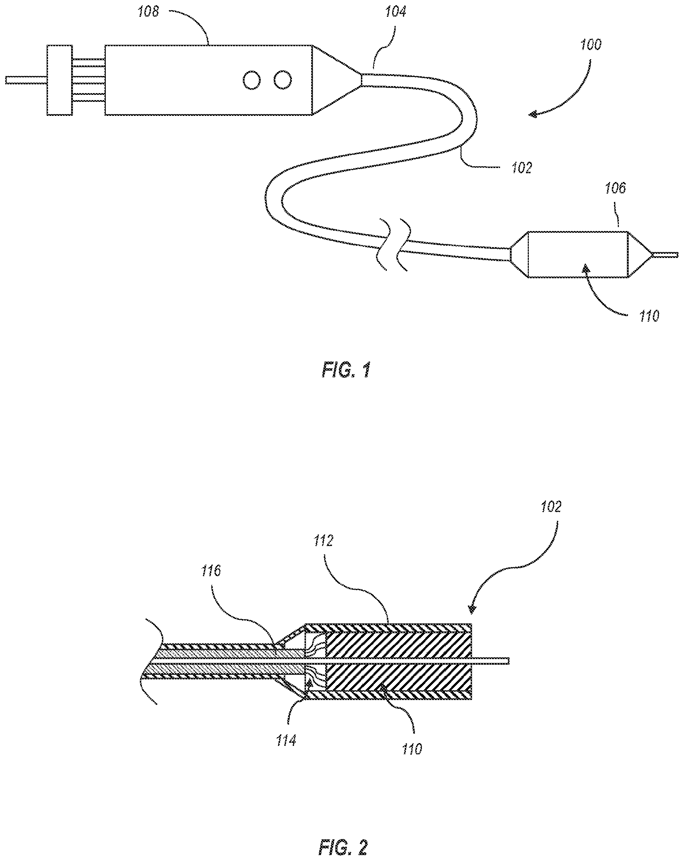

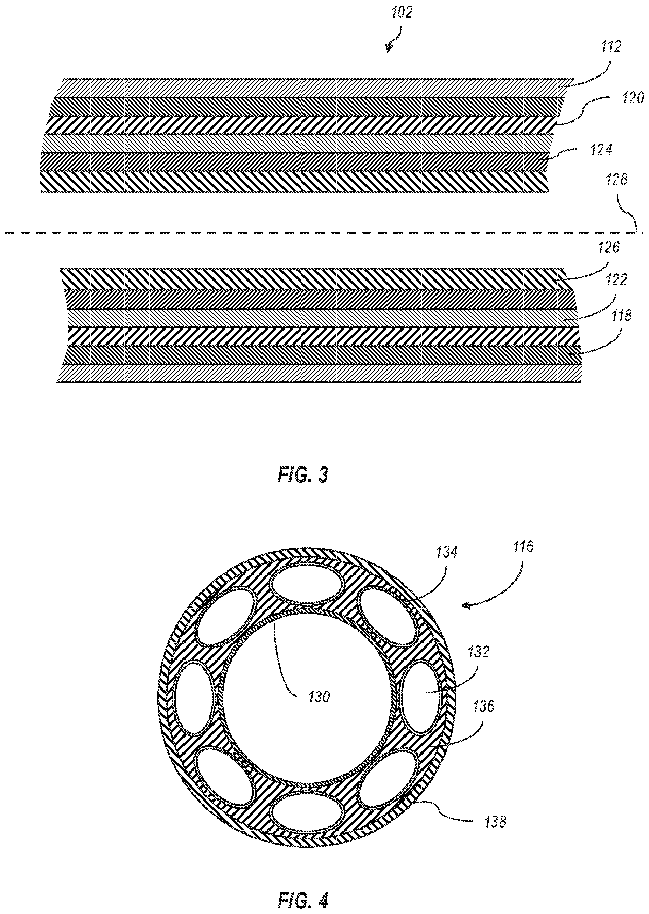

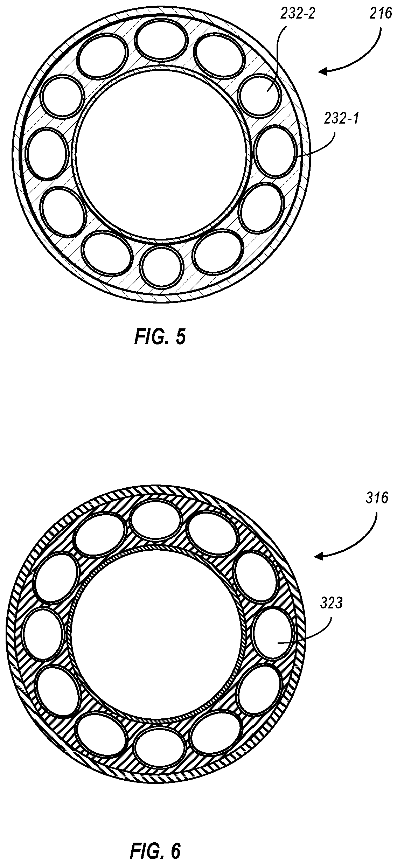

The present disclosure generally relates to manufacturing and using intravascular device delivery systems or other steerable intravascular systems. An intravascular device delivery system allows a medical professional to deliver an intravascular or other medical device to a target location in a patient's body. While the present disclosure will describe intravascular device delivery systems and applications thereof in relation to intravascular procedures in the heart, it should be understood that the devices, systems, and methods described herein may be applicable to other bodily lumens and/or cavities. Additionally, elements described in relation to any arrangement depicted and/or described herein may be combinable with elements described in relation to any other arrangement depicted and/or described herein. For example, any element described in relation to the arrangement depicted in FIG. 3 may be combinable with any element of the arrangement described in FIG. 4, and any element described in relation to the arrangement described in FIG. 5 may be combinable with any element of the arrangement depicted in FIG. 2.

An intravascular device delivery system may include a flexible elongated member that has a distal end and a proximal end. A handle may be connected to a proximal end of the elongated member to allow a user, such as a medical professional and/or clinician, to control one or more movements of the elongated member. An intravascular device may be positioned at and/or connected to the distal end of the elongated member.

The intravascular device delivery system may be configured to deliver an intravascular device that is controlled and/or retained in the elongated member with a plurality of tension members. The tension members may extend from the handle, through at least a portion of the elongated member in a longitudinal direction to the intravascular device. In some arrangements, the tension members connect to the intravascular device to retain the intravascular device in the elongated member during movement of the intravascular device delivery system through the patient's anatomy. In other arrangements, the tension members connect to the intravascular device to actuate one or more moveable and/or deformable portions of the intravascular device, such as a clip used to secure the intravascular device at a target location in the body.

The handle may include one or more controls (e.g., a knob, a button, a lever, or other controls) that may move at least one part of the intravascular device delivery system relative to another. For example, the handle may include one or more controls for moving at least one element of the elongated member relative to another element of the elongated member. The handle may move an inner element relative to an outer element of the elongated member in a proximal direction, in a distal direction, in a rotational direction, or combinations thereof.

FIG. 1 illustrates a schematic representation of an intravascular device delivery system 100. The system 100 may include an elongated member 102 having a proximal end 104 and a distal end 106. One or more handles 108 may be connected to the proximal end 104 of the elongated member 102. An intravascular device 110 may be positioned at and/or connected to the distal end 106.

The elongated member 102 may be flexible, allowing the elongated member 102 to traverse a patient's tortuous vasculature or other anatomy. In some procedures, the elongated member 102 may deliver the intravascular device 110 to a target location in the patient's body, such as delivering a heart valve repair device to the heart. In other procedures, the system 100 and elongated member 102 may be provided without an intravascular device 110 at the distal end 106 such that the system may recapture, reposition, or otherwise move an intravascular device previously positioned in the patient's body.

The elongated member 102 of the system 100 may include one or more elements therein. An element of the elongated member 102 may include a catheter, a guidewire, a sheath, a drive cable, other tubular and/or solid element, or combinations thereof. In some arrangements an element of the elongated member 102 may extend an entire length of the elongated member 102 from a proximal end 104 to a distal end 106 of the elongated member 102. In other arrangements, an element of the elongated member 102 may have a length less than the entire length of the elongated member 102. For example, an element may provide support to the elongated member 102 from the proximal end 104 toward the distal end 106 without continuing the entire length to the distal end 106.

FIG. 2 illustrates a schematic detail cross-sectional side view of the distal end 106 of the elongated member 102 of FIG. 1. The intravascular device 110 is positioned radially within an outer sleeve 112 of the elongated member 102. The intravascular device 110 is carried by the elongated member 102 through the patient's anatomy to the target location in the body. At the target location, the intravascular device 110 may be urged in a distal longitudinal direction such that the intravascular device 110 is no longer radially constrained by the outer sleeve 112. In other arrangements, the outer sleeve 112 may be retracted in a proximal longitudinal direction to allow the intravascular device 110 to deploy radially. In yet other arrangements, the intravascular device 110 is deployed through a combination of the intravascular device 110 moving relative to the elongated member 102 and the outer sleeve 112 moving relative to the remainder of the elongated member 102.

Prior to and/or during deployment of the intravascular device 110, the intravascular device 110 may be retained within the outer sleeve 112 (e.g., the distal longitudinal movement of the intravascular device 110 may be limited) by a plurality of tension members 114 connected to the intravascular device 110. The tension members 114 may be directed to the intravascular device 110 through a multilumen catheter 116 and connect to both a handle and to the intravascular device 110 to transmit tension from the handle to the intravascular device 110. In some arrangements, the tension members 114 are also used to collapse the intravascular device 110 during loading into the outer sleeve 112. During loading, high forces (i.e., up to 445 Newtons (100 pounds of force)) may be generated and additional tension members 114 may distribute the force more to reduce the likelihood of any one of the tension members 114 failing.

FIG. 3 is a side partial cutaway view of part of the elongated member 102 of the intravascular device delivery system 100 of FIG. 1. In some arrangements, the elongated member 102 may include an outer sleeve 112, a steerable guide catheter 118, a steerable catheter 120, a compression coil 122, and an inner catheter 124. Any of the steerable guide catheter 118, steerable catheter 120, and the inner catheter 124 in the elongated member 102 may be a multilumen catheter. The guidewire lumen 126 may be positioned radially within the inner catheter 124 and parallel to a longitudinal axis 128 of the elongated member 102. One or more of the elements of the elongated member 102 may be longitudinally moveable relative to one another. In some arrangements, at least one of the elements of the elongated member is longitudinally fixed relative to another. For example, the inner catheter 124 and compression coil 122 may be longitudinally fixed relative to one another at a distal end of the inner catheter 124 and compression coil 122.

Additionally, one or more elements of the elongated member 102 may be rotationally moveable relative to one another. In some arrangements, at least one of the elements of the elongated member is rotationally fixed relative to another. For example, the outer sleeve 112 and steerable guide catheter 118 may be rotationally fixed relative to one another at an intermediate position between the proximal end and the distal end of the outer sleeve 112.

The multilumen catheter will, during delivery of the intravascular device through the vasculature of the patient, deflect. The length of the tension members relative to one another will change during deflection. For example, tension members which are positioned proximal the inside of a curve will have a shorter distance than those running on the outside of a curve. It may be desirable to keep the diameter of the multilumen catheter as small as possible. A small diameter multilumen catheter may position the tension members 114 closer to the longitudinal axis 128 of the elongated member 102, thereby reducing the relative displacement during deflection.

FIG. 4 illustrates one example of a transverse cross-sectional view of a multilumen catheter 116, not forming part of the claimed invention. 116. The multilumen catheter 116 includes an inner liner 130. The inner liner 130 may include polytetrafluoroethylene (PTFE) or any other lubricous material. A plurality of lumens 132 formed of lubricous tubing (i.e. Polyimide) are placed around the inner liner 130 (in this case 8 lumens). The lumens 132 are held together by a thin braid 134 or coil over the lumens and are covered by a polymeric jacket 138 to form the wall 136 of the multilumen catheter 116.

The thin walled lumens 132 placed in the catheter wall 136 may deform during the delivery process and the deflection of the elongated member. The below calculations show that the lumen ratio of open lumens 132 to catheter wall 136 may be greater than 50%. The calculation is based on a catheter wall without the inner liner 130, the braid 134, and an outer jacket 138. Adding the inner liner 130, the braid 134, and the outer jacket 138 into the calculation may yield a lumen ratio of more than 40%. Tension members may be positioned within the lumens 132 to convey and direct the tension members through the elongated member between the handle and the intravascular device.

FIG. 5 illustrates an example of a multilumen catheter 216, in accordance with the invention, with nine elliptical lumens 232-1 and three round lumens 232-2 for a total of twelve lumens. Calculations for the multilumen catheter 216 shown in FIG. 5 are set forth in Table 1 below:

**TABLE 1**

| | | a | b | d | A | C |
|---|---|---|---|---|---|---|
| Circumference of an ellipse | $C = 2\pi\sqrt{\dfrac{a^2 + b^2}{2}}$ | 0.006 | 0.007875 | | | 0.0439 |
| Circumference of a circle | $C = \pi d$ | | | 0.014 | | 0.0439 |
| Cross sectional area of an ellipse | $A = \pi\,a\,b$ | | | | 0.000148 | |
| Cross sectional area of a circle | $A = \pi\,\dfrac{d^2}{4}$ | | | | 0.000154 | |

| | | | | |
|---|---|---|---|---|
| Total Lumens Area | | 3 round 0.000462 | 9 ellipse 0.001332 | Area total **0.001794** |
| Wall Cross Section without liner, braid and jacket | | ID 0.056 | OD 0.084 | Area **0.003079** 9 |
| **Ratio** Lumen / Wall without liner, braid and jacket | | | | **58.27%** |
| Wall Cross Section with liner, braid and jacket | | 0.054 | 0.09 | **0.004072** |
| **Ratio** Lumen / wall with liner, braid and jacket | | | | **44.06%** |

FIG. 6 illustrates another example of a multilumen catheter 316, not forming part of the claimed invention, with twelve elliptical lumens 323. Calculations for the multilumen catheter 316 shown in FIG. 6 are set forth in Table 2 below:

**TABLE 2**

| | | a | b | d | A | C |
|---|---|---|---|---|---|---|
| Circumference of an ellipse | $C = 2\pi\sqrt{\dfrac{a^2 + b^2}{2}}$ | 0.006 | 0.007875 | | | 0.0439 |
| Circumference of a circle | $C = \pi d$ | | | 0.014 | | 0.0439 |
| circle Cross sectional area of an ellipse | $A = \pi\,a\,b$ | | | | 0.000148 | |
| Cross sectional area of a circle | $A = \pi\,\dfrac{d^2}{4}$ | | | | 0.000154 | |
| Total Lumens Area | | | 12 ellipse 0.001776 | | Area total **0.001776** | |

(continued)

|  |  |  | a | b | d | A | C |
|---|---|---|---|---|---|---|---|
| Wall Cross Section without liner, braid and jacket | | ID 0.056 | | OD 0.084 | | Area **0.003079** | |
| **Ratio** Lumen / Wall without liner, braid and jacket | | | | | | **57.68%** | |
| Wall Cross Section with liner, braid and jacket | | 0.054 | | 0.09 | | **0.004072** | |

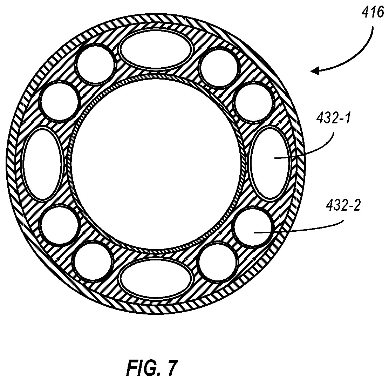

FIG. 7 illustrates another example of a multilumen catheter 416, in accordance with the invention, with a four elliptical lumens 432-1 and eight round lumens 432-2 for a total of twelve lumens.

An intravascular device delivery system according to the present disclosure may allow delivery of larger intravascular devices and/or through smaller bodily conduits. The intravascular device delivery system may allow for new and/or improved procedures with less risk to the patient and greater ease of operation to the medical professional.

The articles "a," "an," and "the" are intended to mean that there are one or more of the elements in the preceding descriptions. The terms "comprising," "including," and "having" are intended to be inclusive and mean that there may be additional elements other than the listed elements. Additionally, it should be understood that references to "one arrangement" or "an arrangement" are not intended to be interpreted as excluding the existence of additional arrangements that also incorporate the recited features.

The described arrangements are to be considered as illustrative and not restrictive. The scope of the disclosure is, therefore, indicated by the appended claims rather than by the foregoing description.

**Claims**

1.  An intravascular device delivery system (100), the system (100) comprising:
    an elongated member (102) with a proximal end (104), a distal end (106), and a longitudinal axis (128) therebetween, the elongated member (102) being configured to have an intravascular device (110) positioned at and/or connected to the distal end (106), the elongated member (102) including:

    a multilumen catheter (116) having a catheter wall (136) having an outer diameter (OD), an internal diameter (ID), and with a plurality of lumens (232-1, 232-2, 432-1, 432-2) formed within the catheter wall (136), the plurality of lumens (132) extending from the proximal end (104) of the elongated member (102) to the distal end (106) of the elongated member (102),
    wherein at least one of the lumens (232-1, 432-1) of the plurality of lumens has an elliptical cross-section and at least one other lumen (232-2, 432-2) of the plurality of lumens has a round cross-section, and
    wherein a lumen ratio, equal to the percentage of the cross sectional area of the catheter wall (136), in a plane perpendicular to the longitudinal axis (128), between the outer diameter (OD) and the inner diameter (ID), that is taken up by the combined cross-sectional areas of the plurality of lumens (232-1, 232-2, 432-1, 432-2), is greater than 30%.

2.  The system (100) of claim 1, wherein the lumen ratio is greater than 40%.

3.  The system (100) of claim 2, wherein the lumen ratio is greater than 50%.

4.  The system (100) of claim 3, wherein at least one of the plurality of lumens (132) is reinforced.

5.  The system (100) of claim 4, wherein the catheter wall (136) is formed in part of an outer jacket (138), the outer jacket (138) contributing to the cross sectional area of the catheter wall (136).

6.  The system (100) of claim 4, wherein the lumens (132) are held together by a braid (134) or coil over the lumens (132) and are covered by a polymeric jacket (138) to form the wall (136) of the multilumen catheter (116).

7. The system (100) of claim 5 or claim 6, wherein the multilumen catheter (116) includes an inner liner (130), the inner liner (130) contributing to the cross sectional area of the catheter wall (136).

8. The system (100) of any of claims 1 to 7, wherein the multilumen catheter has nine elliptical lumens and three round lumens for a total of twelve lumens formed within the catheter wall.

9. The system (100) of any of claims 1 to 7, wherein the multilumen catheter has four elliptical lumens and eight round lumens for a total of twelve lumens formed within the catheter wall.

10. The system (100) of claim 1, wherein the elongated member (102) includes a compression coil (122).


**Patentansprüche**

1. Abgabesystem für eine intravaskuläre Vorrichtung (100), wobei das System (100) umfasst:
ein langgestrecktes Element (102) mit einem proximalen Ende (104), einem distalen Ende (106) und einer Längsachse (128) dazwischen, wobei das langgestreckte Element (102) konfiguriert ist, um eine intravaskuläre Vorrichtung (110) aufzuweisen, die an dem distalen Ende (106) positioniert und/ oder mit diesem verbunden ist, wobei das langgestreckte Element (102) umfasst:

einen Multilumenkatheter (16) mit einer Katheterwand (136), die einen Außendurchmesser (OD), einen Innendurchmesser (ID) und eine Mehrzahl von Lumina (232-1, 232-2, 432-1, 432-2) aufweist, die innerhalb der Katheterwand (136) ausgebildet sind, wobei sich die Mehrzahl von Lumina vom proximalen Ende (104) des langgestreckten Elements (102) zum distalen Ende (106) des langgestreckten Elements (102) erstreckt, wobei mindestens eines der Lumina (232-1, 432-1) aus der Mehrzahl von Lumina einen elliptischen Querschnitt aufweist und mindestens ein anderes Lumen (232-2, 432-2) aus der Mehrzahl von Lumina einen runden Querschnitt aufweist, und
wobei ein Lumenverhältnis, das gleich dem Prozentsatz der Querschnittsfläche der Katheterwand (136) in einer zur Längsachse (128) senkrechten Ebene zwischen dem Außendurchmesser (OD) und dem Innendurchmesser (ID) ist, der von den kombinierten Querschnittsflächen der Mehrzahl von Lumina (232-1, 232-2, 432-1, 432-2) eingenommen wird, größer als 30% ist.

2. System (100) nach Anspruch 1, bei dem das Lumenverhältnis größer als 40% ist.

3. System (100) nach Anspruch 2, bei dem das Lumenverhältnis größer als 50% ist.

4. System (100) nach Anspruch 3, bei dem mindestens eines der Mehrzahl von Lumina (132) verstärkt ist.

5. System (100) nach Anspruch 4, bei dem die Katheterwand (136) zum Teil aus einem Außenmantel (138) gebildet ist, wobei der Außenmantel (138) zur Querschnittsfläche der Katheterwand (136) beiträgt.

6. System (100) nach Anspruch 4, wobei die Lumina (132) durch ein Geflecht (134) oder eine Wicklung über den Lumina (132) zusammengehalten werden und von einem Polymermantel (138) bedeckt sind, um die Wand (136) des Multilumenkatheters (116) zu bilden.

7. System (100) nach Anspruch 5 oder Anspruch 6, bei dem der Multilumenkatheter (116) eine Innenauskleidung (130) aufweist, wobei die Innenauskleidung (130) zur Querschnittsfläche der Katheterwand (136) beiträgt.

8. System (100) nach einem der Ansprüche 1 bis 7, bei dem der Multilumenkatheter neun elliptische Lumina und drei runde Lumina aufweist, so dass insgesamt zwölf Lumina in der Katheterwand gebildet sind.

9. System (100) nach einem der Ansprüche 1 bis 7, bei dem der Multilumenkatheter vier elliptische Lumina und acht runde Lumina aufweist, so dass insgesamt zwölf Lumina in der Katheterwand ausgebildet sind.

10. System (100) nach Anspruch 1, wobei das langgestreckte Element (102) eine Kompressionsspule (122) umfasst.

**Revendications**

1. Système de délivrance de dispositif intravasculaire (100), le système (100) comprenant :
un élément allongé (102) présentant une extrémité proximale (104), une extrémité distale (106), et un axe longitudinal (128) entre celles-ci, l'élément allongé (102) étant configuré pour présenter un dispositif intravasculaire (110) positionné au niveau de et/ou relié à l'extrémité distale (106), l'élément allongé (102) comprenant :

   un cathéter à lumières multiples (116) présentant une paroi de cathéter (136) présentant un diamètre extérieur (OD), un diamètre intérieur (ID), et avec une pluralité de lumières (232-1, 232-2, 432-1, 432-2) formées à l'intérieur de la paroi de cathéter (136), la pluralité de lumières s'étendant depuis l'extrémité proximale (104) de l'élément allongé (102) vers l'extrémité distale (106) de l'élément allongé (102),
   dans lequel au moins une des lumières (232-1, 432-1) de la pluralité de lumières présente une section transversale elliptique et au moins une autre lumière (232-2, 432-2) de la pluralité de lumières présente une section transversale ronde, et
   dans lequel une proportion de lumières, égale au pourcentage de l'aire de section transversale de la paroi du cathéter (136), dans un plan perpendiculaire à l'axe longitudinal (128), entre le diamètre extérieur (OD) et le diamètre intérieur (ID), qui est prise par les aires de section transversale combinées de la pluralité de lumières (232-1, 232-2, 432-1, 432-2), est supérieure à 30 %.

2. Système (100) selon la revendication 1, dans lequel la proportion de lumière est supérieure à 40 %.

3. Système (100) selon la revendication 2, dans lequel la proportion de lumière est supérieure à 50 %.

4. Système (100) selon la revendication 3, dans lequel au moins une de la pluralité de lumières (132) est renforcée.

5. Système (100) selon la revendication 4, dans lequel la paroi de cathéter (136) est formée en partie d'une chemise extérieure (138), la chemise extérieure (138) contribuant à l'aire de section transversale de la paroi de cathéter (136).

6. Système (100) selon la revendication 4, dans lequel les lumières (132) sont maintenues ensemble par une tresse (134) ou une bobine sur les lumières (132) et sont recouvertes par une chemise polymère (138) pour former la paroi (136) du cathéter à lumières multiples (116).

7. Système (100) selon la revendication 5 ou la revendication 6, dans lequel le cathéter à lumières multiples (116) inclut un revêtement intérieur (130), le revêtement intérieur (130) contribuant à l'aire de section transversale de la paroi de cathéter (136).

8. Système (100) selon l'une quelconque des revendications 1 à 7, dans lequel le cathéter à lumières multiples présente neuf lumières elliptiques et trois lumières rondes pour un total de douze lumières formées à l'intérieur de la paroi de cathéter.

9. Système (100) selon l'une quelconque des revendications 1 à 7, dans lequel le cathéter à lumières multiples présente quatre lumières elliptiques et huit lumières rondes pour un total de douze lumières formées à l'intérieur de la paroi de cathéter.

10. Système (100) selon la revendication 1, dans lequel l'élément allongé (102) inclut une bobine de compression (122).

FIG. 1

FIG. 2

*FIG. 3*

*FIG. 4*

FIG. 5

FIG. 6

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050277876 A
- US 20040049207 A
- WO 2016022797 A
- US 20040147826 A
- US 5472423 A
- US 20070060997 A
- WO 2012020521 A
- WO 2013006282 A
- EP 2529701 A